# EUROPEAN PATENT APPLICATION

(11) **EP 2 068 149 A1**
(43) Date of publication of application: **10.06.2009**
(21) Application number: 07023543.7
(22) Date of filing: 05.12.2007
(51) Int. Cl.: G01N 33/569, C12Q 1/37

(54) **Method for determining the toxicity of a toxin or a toxoid**

(71) Applicant: Paul-Ehrlich-Institut Bundesamt für Sera und Impfstoffe, 63225 Langen (DE)
(72) Inventor: The designation of the inventor has not yet been filed
(74) Representative: Forrester, Simon Joseph

(57) **Abstract**

The invention relates to a method for determining the toxicity of a toxin or toxoid that comprises a first subunit for binding to a receptor molecule, and a second subunit for mediating toxicity by catalyzing a reaction of a substrate, wherein the first subunit and the second subunit are separable from each other. According to the invention, the method comprises the steps of: incubating a sample solution containing a toxin or a toxoid with a receptor molecule under conditions that allow for the first subunit of the toxin or toxoid to bind to the receptor molecule, separating the second subunit from the first subunit that is bound to the receptor molecule; incubating the second subunit with a substrate molecule for the second subunit under conditions that allow for the second subunit to catalyze a reaction of the substrate molecule to form a detectable product; and detecting the presence of the detectable product.

## Description

The invention relates to a method for determining the toxicity of a toxin, in particular for determining the toxicity of a toxoid, as well as to a kit for determining the toxicity of a toxin or a toxoid. Furthermore, the invention relates to the use of such a method and such a kit for analyzing the toxicity of a vaccine.

Toxins, in particular bacterial toxins, are used for the production of vaccines. For this purpose, these toxins are usually detoxified using formaldehyde. The product resulting from this process is referred to as toxoid, which is inactive with respect to its toxic activity but is still immunogenic and allows for the production of specific antibodies against it.

In order to exclude that the vaccine containing the toxoid still exhibits toxicity for the individual that is to be vaccinated, each produced toxoid batch needs to undergo testing to ensure that the vaccine does not exhibit any toxicity.

This kind of testing is generally performed with laboratory animals, in particular with guinea pigs. For each batch, testing is usually performed with between five to 15 guinea pigs over a time period of several weeks. In the case that one or several animals die during the testing period and their cause of death cannot directly be linked to the effect of the toxin tested for, the experiment needs to be repeated, which is very time consuming. In addition, all animals need to be killed after the observation period even if they do not develop any symptoms related to the administered toxoid, which raises ethical questions.

Accordingly, the problem underlying the present invention was to provide a means of testing the toxicity of a toxin or of a toxoid that can be used to produce a vaccine, which both yields a result within a relatively short time and circumvents the need of using animals for testing.

Approaches for solving this problem have already been described in the state of the art. For example, Kegel et al. (Toxicology In Vitro 21 (2007) 1641-1649) describe an *in vitro* assay for detection of tetanus neurotoxin activity using antibodies for recognizing the proteolytically generated cleavage product. The inventors have surprisingly found, however, that the enzymatic assay described therein does not allow for unequivocally testing for the presence of toxins in a vaccine. Specifically, the inventors were able to show that tetanus toxoid contains a specific synaptobrevin cleaving activity which does not correlate with the *in vivo* toxicity of the vaccine. Therefore, the *in vitro* toxicity test described by a Kegel et al. might lead to results suggesting the presence of a toxin in the vaccine, although an *in vivo* toxicity cannot be shown in an animal experiment.

This residual enzyme activity is caused by toxoid molecules which still contain an intact enzymatic activity, but are not able to bind to the target molecule of the host cell. From this, the inventors have drawn the conclusion that a test for toxicity needs to comprise a test for binding of the toxoid in addition to a test for the enzymatic activity of the toxin.

Therefore, in comparison to methods which are solely based on the analysis of the catalytic activity of a toxin, the present method is significantly better suited to replace animal testing. The likelihood of obtaining incorrect results stemming from toxoid molecules that have remained to be catalytically active but are not able to bind to the target molecule anymore is significantly reduced. Due to the combined testing of two characteristic features of toxins, namely binding and catalytic activity, the *in vivo* activity of toxins is reflected better. In addition, the invention allows measuring residual toxicity in toxoids that still exhibit enzymatic activity, but are at the time not toxic in *in vivo* experiments.

### Description of the invention

The problem underlying the present invention is solved by a method for determining the toxicity of a bacterial toxin or for determining whether a toxoid derived from such a bacterial toxin still exhibits toxicity. The toxin or toxoid to be analyzed with the present method comprises or consists of two parts, namely a first subunit or a first polypeptide chain for binding to a receptor molecule, and a second subunit or a second polypeptide chain with enzymatic activity for causing toxicity by catalyzing a reaction involving a substrate, e.g. by cleaving a cellular substrate of the host organism or by ADP ribosylating a substrate molecule of the host organism. Toxins which fulfill these criteria are e.g. the members of the class of AB toxins. In other words, the method of the invention can be used for any protein (e.g. toxin or toxoid), for which the binding function can be functionally separated from the enzymatic function of the protein.

It is noted that the term "receptor molecule" used herein is not meant to refer only to a receptor that is part of a signaling cascade, but refers to any molecule that allows for a specific attachment of the toxin or toxoid. In particular, the term "receptor molecule" can refer to any molecule that allows for a specific attachment of the toxin or toxoid to a cell or a molecule of the host organism, like a secreted protein, a protein on the surface of a cell, or lipids or glycolipids like gangliosides.

According to the invention, the method comprises or consists of the following four steps:
- In a first step, a toxin or toxoid is incubated with a receptor molecule under conditions that allow for the first subunit of the toxin or toxoid to specifically bind to the receptor molecule.
- Upon binding of the first subunit to the receptor molecule, the second subunit is separated from the first subunit in a second step.
- In a third step, the separated second subunit is incubated with a substrate molecule for the second subunit under conditions that allow for the second subunit to catalyze a reaction involving the substrate molecule, thereby forming a detectable product.
- This detectable product is then detected in a fourth step, whereby the detection of such a product indicates the presence of a toxic activity, whereas the absence of a detectable product indicates the absence of a toxic activity.

In a preferred embodiment of the invention, the receptor molecule and/or the substrate molecule used in the method are/is immobilized on a solid support, such as a Petri dish, a microtiter plate, a nitrocellulose membrane, or a bead. Due to immobilization of the receptor molecule, the second subunit can easily be separated from the first subunit, as the first subunit remains bound to the immobilized receptor molecule.

After binding of the receptor molecule and/or the substrate molecule on a solid support, it is preferred to block all available binding sites on the solid support with e.g. BSA, casein, skimmed milk, or other suited proteins.

Alternatively, instead of using a substrate molecule that has been attached to a solid support, it is also possible to perform the enzymatic reaction in solution using a dissolved substrate.

In case the first subunit and the second subunit are connected to each other via S-S bridges, the separation of the second subunit from the first subunit is preferably performed using a reducing agent, such as TCEP, DTT, and/or mercaptoethanol. In other embodiments, proteases may be used for the physical separation of the first and the second subunit from each other, in particular when both the first and the second subunit are initially located on the same polypeptide chain.

The present method can be used for all toxins or toxoids that contain at least two subunits, of which one subunit is responsible for binding to a molecule of the host organism, and of which another subunit mediates the enzymatic activity of the toxin. Toxins that fulfill these criteria are in particular tetanus toxin, botulinum toxin, diphtheria toxin, and pertussis toxin.

For some bacterial toxins, various *in vitro* methods for detecting toxicity are known. In most cases, the detection is based on the cytotoxic effect of the toxins on cell cultures, which can be measured and quantified. Examples for this are the Vero cell test for diphtheria toxin as well as the CHO cell test for pertussis toxin. For toxins which do not display a direct cytotoxic effect, however, the development of a reliable in vitro toxicity test is more complex. In some cases, the fact that many bacterial toxins exhibit an enzymatic component was used for the development of *in vitro* methods for the functional detection of toxins. For example, for tetanus and botulinum neurotoxins, which do not exhibit a direct cytotoxic activity, endopeptidase assays have been described, in which the specific proteolytic activity of these toxins was used for their detection (Kegel et al., Toxicology In Vitro 21 (2007) 1641-1649; Hallis et al. 1996, Journal of Clinical Microbiology 34, 1934-1938; Ekong et al. 1997, Microbiology 143, 3337-3347; Ekong et al. 1997, In: van Zutphen L.F.M. and Balls M. (Hrsg.): Animal Alternatives, Welfare and Ethics. Elsevier Amsterdam, 1039-1044; Wictome et al., 1999, Applied and Environmental Microbiology 65, 3787-3792). But as the enzymatic activity of these toxins does not necessarily correlate with their in vivo toxicity, these methods do not allow for an unequivocal functional detection of the toxicity.

The tetanus neurotoxin consists of two subunits, which are linked to each other via a disulfide bridge. The heavy (H-)chain (100 kDa) is responsible for the internalization of the toxin into neurons, and the light (L-)chain (50 kDa) is a zinc dependent metalloprotease (Schiavo et al. 1992, EMBO Journal 11, 3577-3583) to which a significant role in the pathogenesis of the tetanus disease has been ascribed. After internalization of the toxin into the target cell, the light chain of the tetanus toxin catalyzes a specific cleavage of the protein synaptobrevin, which is also known as vesicle associated membrane protein (VAMP) (Link et al., 1992, Biochemical and Biophysical Research Communications 189, 1017-1023; Schiavo et al., 1992, Nature 359, 832-835). Through this cleavage, the release of inhibitory neurotransmitters is impeded, which leads to the muscle spasm characteristic for the tetanus disease.

Synaptobrevin is found in neuronal tissues of all vertebrates. It is linked to the membrane of synaptic vesicles and forms a functional unit with the plasma membrane associated protein syntaxin and SNAP-25. The SNARE complex formed by all of these three proteins connects the vesicle membrane and the plasma membrane and plays an essential role in the fusion of both membranes, which precedes the release of neural transmitters into the synaptic cleft. When synaptobrevin is cleaved by the tetanus toxin, the SNARE complex is not able to form and thus, the release of the neurotransmitters is prevented (Humeau et al., 2000, Biochimie 82, 427-446).

Synaptobrevin is expressed in different isoforms. Among these, synaptobrevin-1 (VAMP-1), synaptobrevin-2 (VAMP-2) and cellubrevin (VAMP-3) are the best characterized isoforms which can be cleaved by the tetanus toxin. *In vivo,* synaptobrevin-1 is an important substrate of the tetanus toxin, due to its primary expression in nerve cells of the motoric system, whereas synaptobrevin-2 plays only a minor role *in vivo,* because it is expressed mainly in sensory neurons (Humeau et al., 2000, Biochimie 82, 427-446; Patarnello et al., 1993, Nature 364, 581-582).

In spite of this, synaptobrevin-2 is the best characterized *in vitro* substrate for tetanus neurotoxin. The peptide bond of synaptobrevin-2 that is specifically cleaved by the tetanus toxin lies between the amino acids Gln 76 and Phe 77 (Link et al., 1992, Biochemical and Biophysical Research Communications 189, 1017-1023; Schiavo et al., 1992, Nature 395, 832-835). The only other neurotoxin that is able to cleave synaptobrevin-2 at the same site is botulinum neurotoxin type B (Schiavo et al., 1992, Nature 395, 832-835). Regarding the substrate specificity, it has been reported that tetanus toxin is not able to cleave a short peptide, which is in contrast to most other proteases. The amino acids 28 to 93 of tetanus neurotoxin are regarded to be essential (Yamasaki et al., 1994, Journal of Biological Chemistry 269, 12764-12772), as well as amino acids 33 to 94 (Foran et al., 1994, Biochemistry 33, 15365-15374).

Accordingly, in a preferred embodiment of the invention, the toxin is tetanus toxin and the receptor molecule is selected from the group consisting of ganglioside GT1b, ganglioside GD1b, ganglioside GQ1b, ganglioside GT1a, ganglioside GM1, ganglioside GD1a, ganglioside GD3, and sialic acid (NeuAc)-containing carbohydrates (e.g. NeuAc (or dimers or oligomers thereof), sialyllactose or disyalyllactose, as well as functional parts thereof, including functional homologs of all of the molecules listed above. As used herein, the term "functional" refers to molecules that exhibit an identical or a similar function to the molecule they mimic. Such functional similarity can be tested by a person of skill in the art using known methods.

Furthermore, when the method is used with respect to tetanus toxin, it is preferred that the substrate molecule is selected from the group consisting of synaptobrevin-1 (vesicle associated membrane protein-1, VAMP-1), synaptobrevin-2 (vesicle associated membrane protein-2, VAMP-2), cellubrevin (vesicle associated membrane protein-3, VAMP-3) and functional parts thereof, as well as functional homologs of all of the before mentioned molecules.

When the present method is used with botulinum toxin, it is preferred that the receptor molecule is selected from the group consisting of polysialogangliosides (e.g. ganglioside GT1b or ganglioside GD1a), sialic acid-containing carbohydrates (e.g. sialyllactose), synaptotagmin-I, synaptotagmin-II, and synaptic vesicle protein SV2, or a combination of the aforementioned molecules, as well as functional parts thereof and functional homologs of all of the before mentioned molecules.

With respect to the substrate molecules, a preferred substrate depends on the type of botulinum toxin or toxoid that is to be analyzed for its toxicity. Specifically, the botulinum toxins types B, D, F, and G cleave synaptobrevin (type B cleaves in exactly the same site as tetanus toxin, whereas types D, F, and G each cleave the substrate at different sites), and botulinum toxins A and E cleave the protein SNAP-25, and botulinum toxin type C cleaves both the proteins SNAP-25, and syntaxin.

When the present method is used for pertussis toxin, a preferred receptor molecule is selected from the group consisting of a protein or a glycoprotein receptor (in particular the human T cell receptor (TcR) or a closely associated T cell pertussis toxin receptor (PTx-R)), an oligosaccharide structure alone (e.g. a sialyllactosamin residue), a glycolipid (e.g. ganglioside GD1a), as well as functional parts thereof and including functional homologs of all the before mentioned molecules.

Preferred substrate molecules for pertussis toxin are selected from the group consisting of NAD and inhibitory G-proteins, in particular α-subunits of heterotrimeric inhibitory G-proteins, as well as parts thereof and including functional homologs of all of the before mentioned molecules.

When the present method is used for diphtheria toxin, a preferred receptor molecule is selected from the group consisting of diphtheria toxin receptors (DTR), such as the membrane-anchored heparin-binding EGF-like growth factor (HB-EGF or proHB-EGF, resp.), as well as thereof parts or precursor forms thereof and including functional homologs of all the before mentioned molecules.

Preferred substrate molecules for diphtheria toxin are selected from the group consisting of NAD and elongation factors, in particular the eukaryotic elongation factor 2 (EF-2, or eEF-2), as well as functional parts thereof or functional homologs thereof.

The substrate molecule that is attached to a solid support can be labeled such that upon serving as a substrate with the second subunit, a labeled product is freed which can then be analyzed, e.g. in a fluorescence assay or an ELISA. It is also possible to detect the product of the catalytic reaction of the second subunit with an antibody, for example an antibody that specifically recognizes the newly generated amino- or carboxy-terminus of either of the cleavage fragments, in case the second subunit has cleaving activity. For a second subunit with ADP ribosylating activity, antibodies specifically recognizing the ribosylated product can be used. Alternatively, it is possible to label the substrate molecule NAD, e.g. with radioisotopes, fluorescent moieties, or biotin, and then analyze the reaction product carrying the label (i.e. either nicotinamide or ADP-ribose) using a suitable method, like a fluorescence assay or scintillation counting. Further alternatives are known in the art.

The use of a product specific antibody is preferably accompanied by using a second antibody specifically binding to the first antibody that is conjugated e.g. with biotin, which can be detected and quantified with streptavidin-coupled peroxidase and the substrate TMB using photometry. Alternatively, the second antibody can also be directly conjugated with peroxidase. As an alternative to TMB, also other peroxidase substrates which are converted into colorimetric, fluorescent or chemiluminescent products can be used. Further alternatives are known in the art.

Using an appropriate standard, the values obtained from the detectable product can be quantified.

The problem underlying the present invention is also solved by a kit for determining the toxicity of a bacterial toxin or of a toxoid that is derived from a bacterial toxin. Such a toxin comprises or consists of a first subunit for binding to a receptor molecule and a second subunit for mediating toxicity by catalyzing a reaction of a substrate, wherein the first subunit and the second subunit can be separated both functionally and physically from each other. According to the invention, the kit comprises or consists of a first support on which a receptor molecule for binding to the first subunit of the toxin is immobilized, and a second support on which a substrate for the second subunit molecule is immobilized. It is preferred that the solid support is or comprises a Petri dish, a microtiter plate, a nitrocellulose membrane, and/or a bead. Further features of the kit according to the invention are described with reference to the method of the invention above.

The problem underlying the present invention is also solved by the use of the method as described above as well as by the use of a kit as described above for analyzing the toxicity of a vaccine that comprises at least one toxoid.

The present invention can also be applied to quantify the activity of toxins used for medical or cosmetic reasons, such as botulinum toxin type A ("botox") or type B ("Neurobloc", "Myobloc").

The method and kit as described above can also be used for diagnosing a patient who is suspected of suffering from a disease caused by toxin producing bacteria.

### Figure

The invention is now further described with reference to the figure.

### Figure 1:

### Results from the ganglioside binding assay (A, B), the endopeptidase assay (C, D), and the combined assay (E-G) for tetanus toxin as well as tetanus toxoid

The results show that tetanus toxin (A) as well as tetanus toxoid (B) both are able to bind to gangliosides. Similarly, both tetanus toxin (C) and tetanus toxoid (D) show a synaptobrevin-cleaving activity in the endopeptidase assay. Thus, neither of these methods, if applied separately, is capable of discriminating between toxic and non-toxic samples.

When both methods are functionally linked, however, a clear correlation between in vivo toxicity and assay signal is observed: In the combined assay, only the samples containing active tetanus toxin caused a signal (E), but not the toxoid samples (F). A detection of active toxin, which had been experimentally added to toxoids, was also possible using the combined assay (G).

### EXAMPLES

The present invention is now described in an example with reference to the figure.

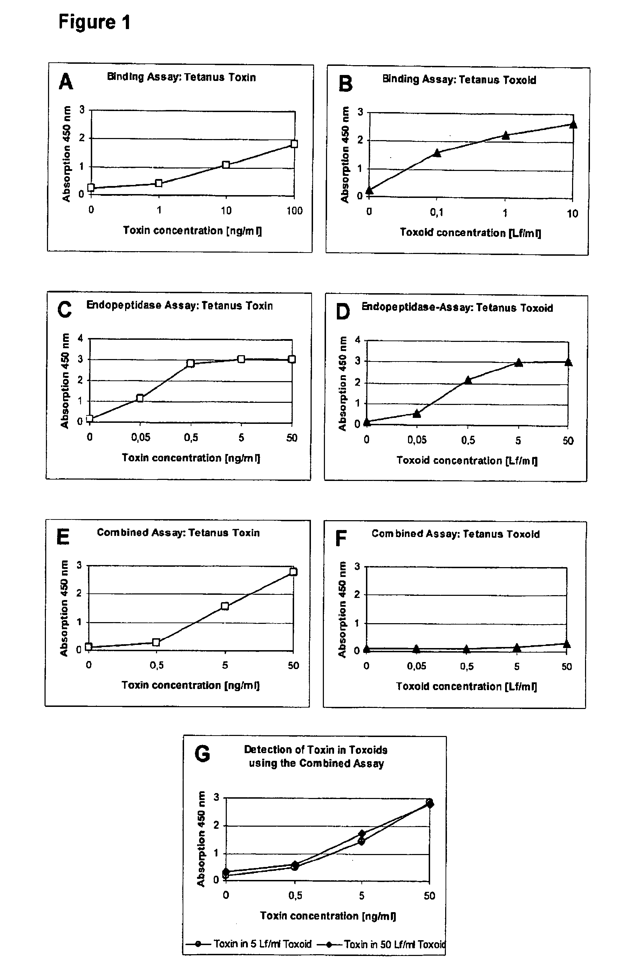

In the following, a method for determining the toxicity of a toxin according to the present invention is described. As a toxin, tetanus toxin was chosen. The results of experiments performed according to the following protocol are shown in figure 1. In order to show the advantages of combining a binding assay with an assay detecting the catalytic activity of a toxin, experiments using only the binding assay, only the catalytic activity assay, and a combined assay were performed.

### I. Gangloside-binding assay

### 1. Coating a microtiter plate with ganglioside GT1b

A stock solution of ganglioside GT1b (1 mg/ml in methanol) was prepared and stored at -20 °C. The stock solution was diluted in methanol or ethanol to a final concentration of 10 µg/ml. Into each well of a MaxiSorp microtiter plate, 100 ul of the diluted GT1b solution were pipetted. Alternatively, 50 µl of a 20 µg/ml GT1b solution were pipetted per well. The solvent was allowed to evaporate at room temperature, immobilizing the receptor molecule GT1b on the microtiter plate. The wells were then washed with 300 µl of PBS/0.05 % Tween-20 for four consecutive times. Blocking was performed for one hour at 37 °C at 250 rpm with 250 µl/well of PBS/3% BSA. The wells were then again washed four times as described above.

### 2. Addition of the toxin or toxoid to be analyzed

The toxin or toxoid to be tested was diluted with PBS/0.05 % Tween-20/3 % BSA to the desired final concentration. Of this toxin or toxoid solution, 100 µl were pipetted into each well of the coated microtiter plates. The microtiter plates were incubated for two hours at 37 °C at 250 rpm or over night at 4 °C. After incubation, the plates were washed four times with 300 µl of PBS/0.05 % Tween-20 per well.

With reference to the results shown in figure 1, the bound material can now either be quantified according to section 3a (binding test only) or can be reduced according to section 3b and then be tested for its enzymatic activity (combined assay of binding test and catalytic activity test).

### 3a Quantification of the materials bound

100 µl of diluted tetanus antisera from rabbits were added to each well and incubated for one hour at 37 °C at 250 rpm. The wells were washed four times with 300 µl/well PBS/0.05 % Tween-20. 100 µl of diluted biotinylated goat-anti-rabbit-antibody was added and incubated for one hour at 37 °C at 250 rpm. The wells were again washed four times with 300 µl PBS/0.05 % Tween-20. Then, 100 µl of a diluted streptavidin-peroxidase was added and incubated for one hour at 37 °C at 250 rpm, followed by washing the wells with 300 µl of PBS/0.05 % Tween-20 four times. 100 µl TMB developing solution was added and incubated at room temperature in the dark. The reaction was stopped using 50 µl/well of a solution of H₂SO₄ of a concentration of 1 mol/l. Absorption was measured at 450 nm against a reference wavelength of 620 nm.

### 3b Reduction of the bound materials for consecutive testing in the enzymatic assay

The wells were washed once with 300 µl of 100 mmol/l PIPES, pH 6.8. 100 µl of 100 mmol/l PIPES, pH 6.8 containing 10 mmol/l TCEP as a reducing agent were added and incubated for 30 minutes at 37 °C at 250 rpm. Thereby, the L-chain of the tetanus toxin or toxoid is separated from the H-chain. The H-chain remains bound to the wells via the immobilized receptor molecule. From each well, 50 µl of the supernatant (containing the L-chain) were transferred to a microtiter plate coated with rSyb2 as a substrate molecule.

### II. Synaptobrevin cleavage assay

### 1. Coating of the microtiter plates with recombinant synaptobrevin-2

rSyb2 (a recombinant protein representing amino acids 1 to 97 of rat synaptobrevin-2 with an amino-terminal histidine tag, Kegel et al., Toxicology In Vitro 21 (2007) 1641-1649) was diluted in PBS to a final concentration of 1.5 µmol/l. Into each well of a MaxiSorp microtiter plate (Nunc), 100 µl of the diluted rSyb2 were pipetted. Incubation was performed for two hours at 37 °C at 250 rpm. Blocking was performed over night at 4 °C in 250 rpm with 250 µl/well PBS/3 % BSA/5 % sucrose/250 µg/ml asolectin. The wells were washed four times with 300 µl PBS/0.05 % Tween-20 and once with 300 µl PBS. Into each well, 50 µl of 100 mmol/l PIPES pH 6.8/5 % sucrose/100 µg/ml asolectin were pipetted.

### 2. Cleavage of the substrate

Into each well, 50 µl of the reduced supernatant from step I 3b were added. In addition, a dilution series of reduced toxin was also pipetted on the same microtiter plate. The plate was then incubated for six hours at 37 °C at 250 rpm, followed by four consecutive washing steps with 300 µl PBS/0.05 % Tween-20.

### 3. Detection of the cleaved fragments

100 µl of a diluted rabbit antibody that specifically recognizes the cleaved product was added to each well (Kegel et al., Toxicology In Vitro 21 (2007) 1641-1649) and incubated over night at 4 °C at 250 rpm. Wells were washed four times with 300 µl PBS/0.05 % Tween-20. 100 µl of diluted biotinylated goat-anti-rabbit antibody (Dianova) were added and incubated for 45 minutes at room temperature at 250 rpm, followed by washing with 300 µl PBS/0.05 % Tween-20 for four times. 100 µl of a diluted streptavidin-peroxidase solution was added and incubated for 45 minutes at room temperature at 250 rpm. After washing for five times with 300 µl PBS/0.05 % Tween-20, 100 µl TMB development solution were added and incubated at room temperature in the dark. The reaction was stopped by adding 50 µl of a 1 mol/l H₂SO₄ solution. Absorption was measured at 450 nm against a reference wavelength of 620 nm.

## Claims

1. Method for determining the toxicity of a toxin or a toxoid that comprises
- a first subunit for binding to a receptor molecule, and
- a second subunit for mediating toxicity by catalyzing a reaction of a substrate, wherein the first subunit and the second subunit are separable from each other, comprising the steps of:
- incubating a toxin or a toxoid with a receptor molecule under conditions that allow for the first subunit of the toxin or the toxoid to bind to the receptor molecule,
- separating the second subunit from the first subunit that is bound to the receptor molecule;
- incubating the second subunit with a substrate molecule for the second subunit under conditions that allow for the second subunit to catalyze a reaction of the substrate molecule to form a detectable product; and
- detecting the presence of the detectable product.

2. The method according to claim 1, wherein the separation of the second subunit from the first subunit is performed with a reducing agent.

3. The method according to claim 1 or 2, wherein the receptor molecule or the substrate molecule is immobilized on a solid support.

4. The method according to claims 1 to 3, wherein the toxin is tetanus toxin, and wherein the receptor molecule is selected from the group consisting of ganglioside GT1b, ganglioside GD1b, ganglioside GQ1b, ganglioside GT1a, ganglioside GM1, ganglioside GD1a, ganglioside GD3, and sialic acid (NeuAc)-containing carbohydrates, and parts thereof, and homologs thereof.

5. The method according to claims 1 to 4, wherein the toxin is tetanus toxin, and wherein the substrate molecule is selected from the group consisting of synaptobrevin-1 (VAMP-1), synaptobrevin-2 (VAMP-2), cellubrevin (VAMP-3), and parts thereof, and homologs thereof.

6. The method according to claims 1 to 3, wherein the toxin is botulinum toxin, and wherein the receptor molecule is selected from the group consisting of ganglioside GT1b, ganglioside GD1a, sialic acid-containing carbohydrates, synaptotagmin-1, synaptotagmin-II, synaptic vesicle protein SV2, and parts thereof, and homologs thereof.

7. The method according to claims 1, 2, 3, or 6, wherein the toxin is botulinum toxin, and wherein the substrate molecule is selected from the group consisting of synaptobrevin-1 (VAMP-1), synaptobrevin-2 (VAMP-2), cellubrevin (VAMP-3), SNAP-25, and Syntaxin, and parts thereof, and homologs thereof.

8. The method according to claims 1 to 3, wherein the toxin is pertussis toxin, and wherein the receptor molecule is selected from the group consisting of protein receptors, glycoprotein receptors, oligosaccharide structures, glycolipids, as well as parts thereof and homologs thereof.

9. The method according to claims 1, 2, 3, or 8, wherein the toxin is pertussis toxin, and wherein the substrate molecule is selected from the group consisting of NAD and inhibitory G-proteins, and parts thereof, and homologs thereof.

10. The method according to claims 1 to 3, wherein the toxin is diphtheria toxin, and wherein the receptor molecule is selected from the group consisting of diphtheria toxin receptors, as well as parts or precursor forms thereof and homologs thereof.

11. The method according to claims 1, 2, 3, or 10, wherein the toxin is diphtheria toxin, and wherein the substrate molecule is selected from the group consisting of NAD and elongation factors, as well as parts and homologs thereof.

12. A kit for determining the toxicity of a toxin or a toxoid, comprising
- a first support on which a receptor molecule for binding to the first subunit is immobilized, and
- a second support on which a substrate for the second subunit molecule is immobilized.

13. The kit according to claim 12, wherein the solid support is a Petri dish, a microtiter plate, a nitrocellulose membrane, or a bead.

14. Use of a method according to claims 1 to 11 for analyzing the toxicity of a vaccine.

15. Use of a kit according to claims 12 or 13 for analyzing the toxicity of a vaccine.
